# EUROPEAN PATENT SPECIFICATION

(11) **EP 1 602 388 B1**
(45) Date of publication and mention of the grant of the patent: **11.11.2009**
(21) Application number: 04425411.8
(22) Date of filing: 03.06.2004
(51) Int. Cl.: A61M 5/14

(54) **Intravenous infusion system with a multiaccess manifold device**
System zur intravenösen Infusion mit Mehrkanalanschlussblock
Système d'injection par voie intraveineuse avec collecteur à accès multiple

(43) Date of publication of application: 07.12.2005
(73) Proprietor: Unicare S.r.L., Frazione Levane 52021 Bucine (AR) (IT)
(72) Inventor: Forino, Luciano, 10100 Torino (IT); Marini, Andrea, 52028 Terranuova Bracciolini (IT)
(74) Representative: Jorio, Paolo

(56) References cited:
- WO-A-03/084585
- DE-A- 3 503 044
- US-A- 6 099 511

## Description

The present invention relates to an intravenous infusion system with a multi-access manifold device. The present invention may be used to particular advantage, though not exclusively, in general anaesthesia and pre- and post-operative intravenous infusion applications.

As is known, in general anaesthesia, massive doses of hypnotic drugs are first administered to induce a sufficiently high degree of anaesthesia in a patient, and smaller doses of the same drugs are subsequently administered to maintain roughly the same degree of anaesthesia throughout the operation.

In addition to hypnotic drugs, analgesics or pain-relieving drugs, myorelaxants for relaxing/paralyzing the muscles, and/or other liquids, such as saline solutions, hypertonic solutions, blood or its derivatives, are normally also administered.

Various systems have been proposed for controlled, selective, continuous administration of various drugs and/or liquids, and which at the same time are quick and easy to use by medical and nursing staff.

One proposed system, for example, comprises a multiple-inlet manifold for controlling infusion of drugs and/or other liquids or solutions into a patient's intravenous feed line.

More specifically, the manifold comprises a casing defining a flow channel communicating hydraulically at one end with a carrier liquid inlet line (for carrying the drugs into the patient's body), and at the other end with an intravenous feed line to the patient.

The manifold also comprises a number of inlet openings formed in the casing and communicating hydraulically with the flow channel.

Each inlet opening can be engaged releasably by a syringe or other appropriate administration device containing a fluid to be administered to the patient.

Each inlet opening is fitted with a one-way valve permitting flow (of the drug or other solution for injection) from the syringe to the flow channel, and preventing flow in the opposite direction.

More specifically, the valve substantially comprises a flexible shutter with a disk-shaped membrane, the periphery of which rests on an annular shoulder defined by the relative inlet opening. In the presence of a fluid pressure directed to hold the shutter against the shoulder, no flow is permitted; and, conversely, in the presence of a fluid pressure in the opposite direction, i.e. detaching the periphery of the shutter from the shoulder, fluid flows into the flow channel.

The intravenous infusion system described above has various advantages.

First of all, it enables connection of a number of syringes, containing different drugs, to one feed line to the patient, and provides for accurately and continuously regulating administration, with no need for repeated connection to the peripheral patient access point.

Moreover, the one-way valves inside the inlet openings prevent fluid backflow from the patient from contaminating the drugs inside the various syringes, and prevent the drug injected by one syringe from flowing into and possibly reacting chemically or coagulating inside an adjacent syringe.

This characteristic is particularly advantageous in the case of high-cost drugs, by enabling use of the surplus drugs left in the syringes at the end of the operation.

An intravenous infusion system of this type, however, also has various disadvantages.

Firstly, because of the particular configuration of the one-way valves, it does not permit high fluid flow, as frequently required during surgical operations or in intensive care.

Secondly, drug administration is frequently accompanied by repeated backflow of blood, because of the low hydrostatic pressure of the infusion system, thus contaminating and preventing reuse of the flow channel of the system.

WO 03/084585 discloses an intravenous system of the prior art.

It is an object of the present invention to eliminate the drawbacks of the known art, and to further improve intravenous infusion systems by making them particularly versatile.

According to the present invention, there is provided an intravenous infusion system as claimed in Claim 1.

A preferred, non-limiting embodiment of the present invention will be described by way of example with reference to the accompanying drawings, in which:
Figure 1 shows an overall view of the intravenous infusion system according to the present invention;
Figure 2 shows a partly sectioned side view of a manifold device of the Figure 1 system;
Figure 3 shows a plan view of the Figure 2 manifold device;
Figure 4 shows a section along line IV-IV in Figure 3, showing a first operating condition of the manifold device;
Figure 5 shows a section along line V-V in Figure 4;
Figure 6 shows the same section as in Figure 4, showing a second operating condition of the manifold device;
Figure 7 shows a section along line VII-VII in Figure 6;
Figure 8 shows a section along line VIII-VIII in Figure 3;
Figure 9 shows a section along line IX-IX in Figure 8;
Figure 10 shows a top plan view of a releasable connecting device of the Figure 1 system;
Figure 11 shows an axial section of the Figure 10 connecting device;
Figure 12 shows a side view of the component parts of the Figure 10 connecting device disconnected;
Figure 13 shows a section along line XIII-XIII in Figure 12;
Figures 14 and 15 show front views of the Figure 10 connecting device when disconnecting the component parts.

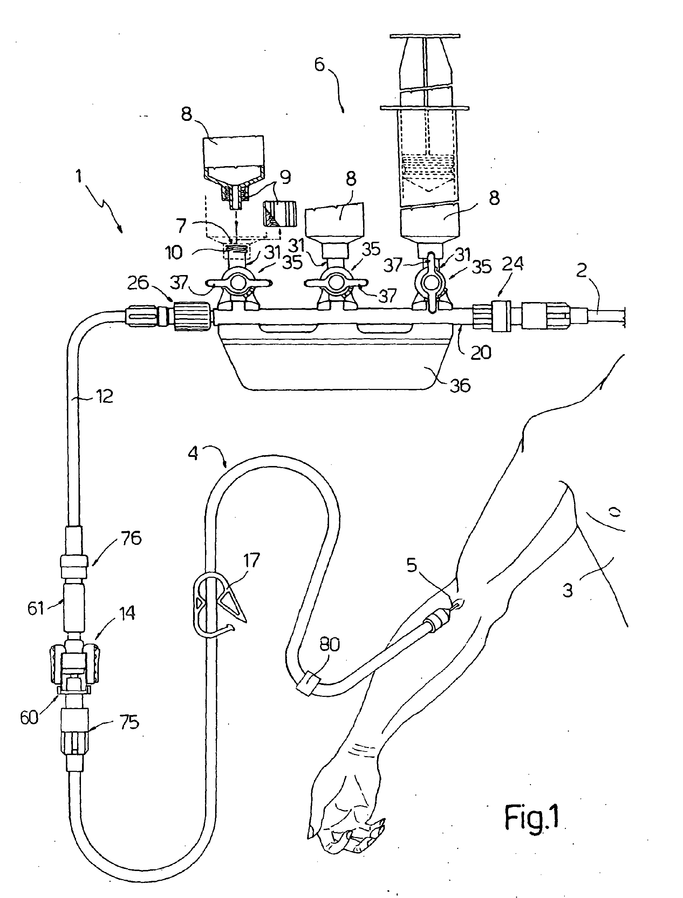

With reference to Figure 1, an intravenous infusion system 1 comprises an inlet line 2, defined by a flexible tube, for a primary parenteral fluid (e.g. a solution containing glucose or lipids) for administration to a patient 3. The primary parenteral fluid is normally contained in plastic bags (not shown), from which it flows continuously by force of gravity or with the aid of an infusion pump (not shown).

System 1 also comprises a patient feed line 4 terminating with an intravenous infusion cannula 5 inserted inside a vein; and a manifold device 6 interposed between and communicating hydraulically with inlet line 2 and patient feed line 4.

Manifold device 6, which is described in detail later on, comprises a number of accesses 7 connectable releasably to respective fluid administration/withdrawal devices 8, in particular syringes. More specifically, each administration/withdrawal device 8 comprises a threaded end portion 9 which screws onto a corresponding threaded portion 10 of an access 7.

Administration/withdrawal devices 8 may contain respective supplementary fluids, drugs or solutions to be administered continuously or extemporaneously to patient 3, and which are fed into patient feed line 4 and injected into patient 3 via cannula 5.

System 1 also comprises a flexible connecting tube 12 connected hydraulically to manifold device 6 and patient feed line 4 by a releasable connecting device 14. More specifically, releasable connecting device 14, which is described in detail later on, provides for quickly and easily separating the part of system 1 containing the drugs hermetically from patient feed line 4.

System 1 also comprises cutoff means 17 located along patient feed line 4 to cut off fluid flow to patient 3 by pinching and so closing the section of the flexible tube defining the feed line.

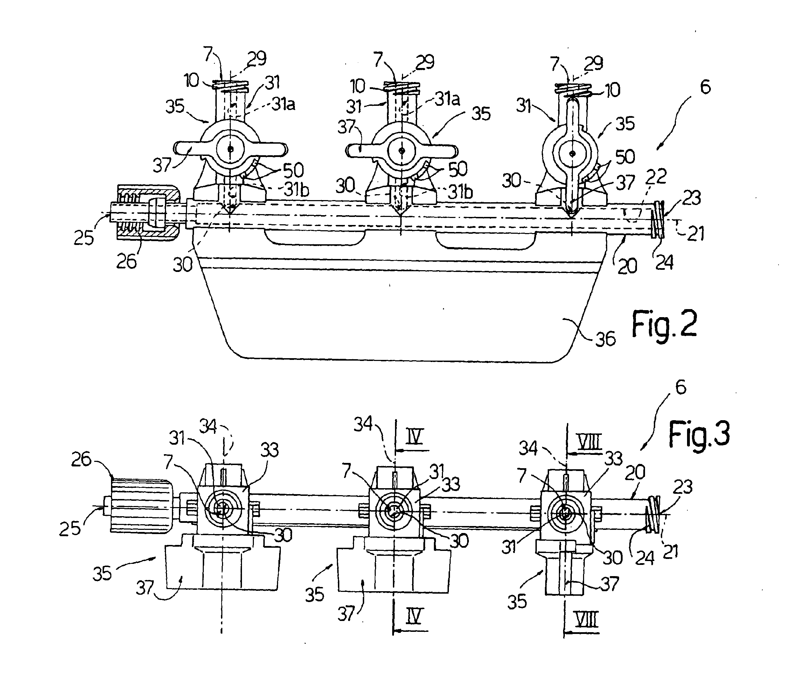

More specifically, and with reference to Figures 2 and 3, manifold device 6 comprises an elongated body 20 having a longitudinal axis 21, and made of transparent, biocompatible, lipid-resistant material, such as copolyester, polysulphone, polyethylene, and other of its derivatives.

Elongated body 20 defines, along longitudinal axis 21, a tubular, circular-section, main fluid flow conduit 22 comprising a first opening 23 connectable hydraulically to inlet line 2 by a first threaded connection 24, and a second opening 25 connectable hydraulically to connecting tube 12 by a second threaded connection 26.

The lateral surface of main conduit 22 has a number of access openings 30, from which respective tubular, circular-section, secondary conduits 31 extend outwards, and in particular along respective axes 29 perpendicular to longitudinal axis 21.

From the lateral surface of main conduit 22, on the opposite side of longitudinal axis 21 to secondary conduits 31, there extends a grip portion 36, which can be gripped by the user to facilitate administration of the fluids contained in administration/withdrawal devices 8, or can be fixed to an appropriate supporting surface.

More specifically, each secondary conduit 31 comprises an open end defining a respective access 7 of manifold device 6, and having threaded portion 10 for receiving a respective administration/withdrawal device 8.

Each secondary conduit 31 also comprises a substantially cylindrical wide portion 33 having an axis 34 perpendicular to axes 21 and 29, and defining a cavity 33a housing a respective regulating valve 35 for regulating fluid flow inside secondary conduit 31. On opposite sides along axis 29, each wide portion 33 communicates with respective open portions 31a, 31b of secondary conduit 31.

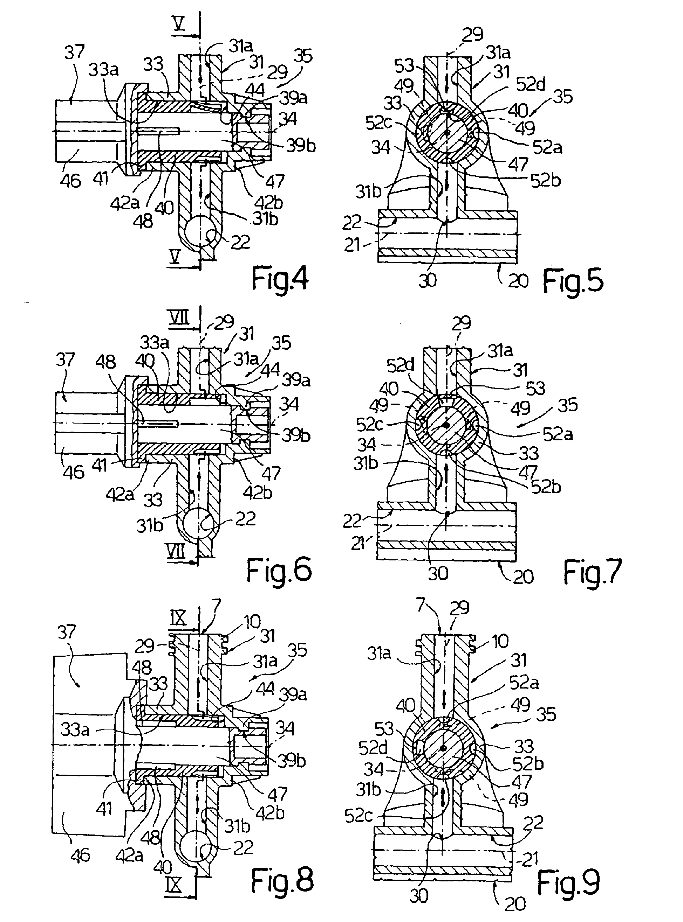

Valves 35 can be set manually by the user to two operating conditions: a first operating condition (Figures 1-3 - the two valves 35 on the left, and Figures 4-7) permitting low, one-way fluid flow from administration/withdrawal device 8 to main conduit 22; and a second operating condition (Figures 1-3 - valve 35 on the right, and Figures 8, 9) permitting high fluid flow, not only from administration/withdrawal device 8 to main conduit 22, but also in the opposite direction, thus operating as two-way valves.

The above characteristic provides for a high degree of versatility of manifold device 6.

That is, in general anaesthesia and pre- and post-operative intravenous infusion applications, manifold device 6 may be used together with a conventional non-return valve 80 located along patient feed line 4 and indicated only schematically for the sake of simplicity. In which case, the first and second operating condition of valves 35 may be used to permit low and high (e.g. emergency) flow respectively from administration/withdrawal devices 8 to the patient, with no risk of fluid backflow from the patient contaminating the drugs or manifold device 6 as a whole.

Alternatively, manifold device 6 may also be used to advantage for taking samples from the patient, by simply setting valves 35 to the second operating condition permitting two-way flow, obviously with no non-return valves downstream from manifold device 6.

More specifically, and with reference to Figures 4-9, each regulating valve 35 comprises a tubular, cylindrical, flexible shutter 40 inserted, coaxially with axis 34, inside wide portion 33 of a respective secondary conduit 31, and interposed between opposite portions 31a and 31b of secondary conduit 31.

Each regulating valve 35 also comprises a selection knob 37 for operating regulating valve 35, and which axially engages wide portion 33 and has a user grip portion 46 at one end.

Each selection knob 37 comprises a shaft 47 projecting from grip portion 46 and fitted externally with shutter 40.

More specifically, grip portion 46 is elongated perpendicularly to axis 34, and extends on diametrically opposite sides of shaft 47 for two-finger operation by the user. Shaft 47 has two outer axial ribs 48 engaging respective complementary grooves 49 formed in the inner lateral surface of shutter 40, so that shaft 47 is angularly integral with shutter 40.

More specifically, shutter 40 adheres to the inner wall of wide portion 33 of a respective secondary conduit 31, and comprises an end flange 41 projecting radially outwards and interposed axially and hermetically between an axial end 42a of wide portion 33 and grip portion 46 of relative selection knob 37. The length of shutter 40 along axis 34 is less than the length of relative wide portion 33, so that a free portion 44 of cavity 33a defined by wide portion 33 is defined at the opposite end to end 42a.

On the opposite open side to grip portion 46 along axis 34, shaft 47 hermetically closes a second end 42b of relative wide portion 33, so that free portion 44 has an annular section defined by the inner wall of wide portion 33 and by shaft 47. Withdrawal of selection knob 37 along axis 34 is prevented by an inner circumferential rib 39a on wide portion 33 engaging an outer annular groove 39b on shaft 47.

Selection knob 37 rotates about axis 34 between a first operating position (Figures 4-7) in which grip portion 46 is parallel to longitudinal axis 21, and a second operating position (Figures 8, 9) in which grip portion 46 is parallel to axis 29. Two projections 50, extending outwards from the lateral surface of wide portion 33 (Figure 2), define respective stops in opposite directions for grip portion 46, so as to limit rotation of the selection knob, between the first and second operating position, to an angle of 90°.

Shutter 40, which is made for example of silicone, comprises, at the opposite end to flange 41, four recesses 52a-52d equally spaced angularly about axis 34. More specifically, recess 52a is diametrically opposite recess 52c, and recess 52b diametrically opposite recess 52d, with respect to axis 34.

Three of the four recesses, 52a-52c, are identical and formed along the outer lateral surface of shutter 40, while the fourth recess, 52d, is formed along the inner lateral surface of shutter 40, i.e. as a continuation of cavity 33a defined by shutter 40, so as to define a thin membrane 53.

Selection knob 37 and shutter 40 are movable integrally about axis 34 so that, in the first operating position of selection 37 corresponding to the first operating condition of valve 35, membrane 53 and recess 52b are aligned respectively with portions 31a, 31b of secondary conduit 31, and only permit low, one-way flow to main conduit 22 (Figures 4-7), and, in the second operating position of selection knob 37 corresponding to the second operating condition of valve 35, recesses 52a and 52c are aligned respectively with portions 31a, 31b of secondary conduit 31 (Figures 8, 9) to permit high, one-way flow in combination with non-return valve 80, and high, two-way flow without non-return valve 80.

More specifically, in the first operating condition of valve 35 (Figures 4, 5), the pressurized fluid fed by administration/withdrawal device 8 into portion 31a of secondary conduit 31 (the direction of which is indicated by the arrow in the drawings) pushes membrane 53 towards opposite recess 52d onto shaft 47, so as to detach the membrane from the lateral edge of the inlet between portion 31a and inner cavity 33a of wide portion 33, and so create a fluid passage into free portion 44, and from this into the opposite portion 31b of secondary conduit 31 through recess 52b.

When valve 35 is in the first operating condition, fluid flow in the opposite direction is prevented (Figures 6, 7).

That is, fluid flows from portion 31b into free portion 44 through recess 52b, but is prevented from flowing from free portion 44 into the opposite portion 31a by membrane 53 being pushed onto the lateral edge of the inlet between portion 31a and inner cavity 33a of wide portion 33.

In the second operating condition of valve 35 (Figures 8, 9), two-way fluid flow is permitted by recesses 52a and 52c being aligned with portions 31a and 31b of secondary conduit 31, and so hydraulically connecting portions 31a and 31b to free portion 44.

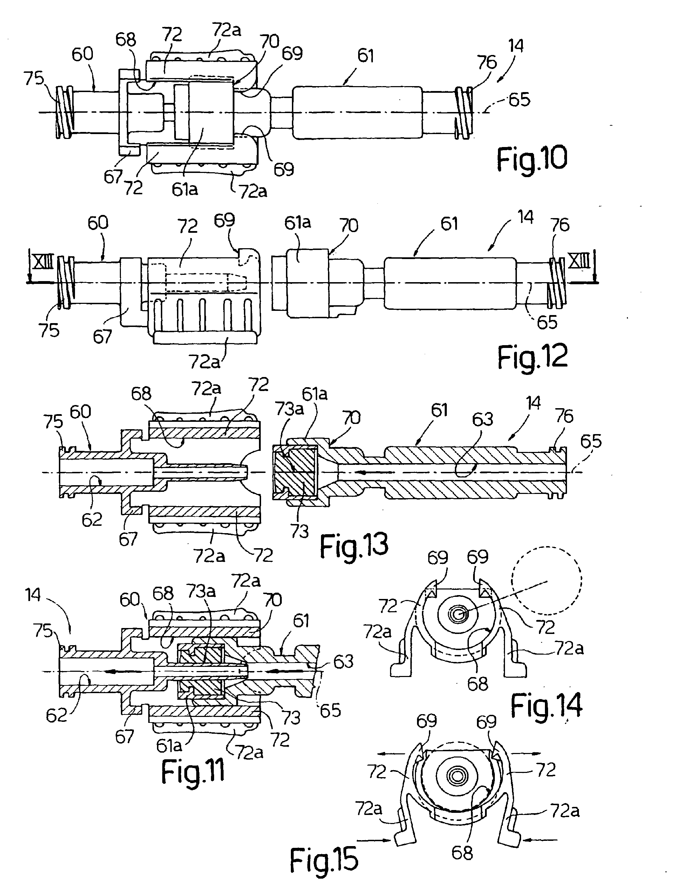

Releasable connecting device 14 will now be described in detail with reference to Figures 10-15.

Releasable connecting device 14 comprises a male member 60 and a female member 61 uniquely compatible with each other and defining an elongated tubular first and second conduit 62, 63 extending along a common axis 65, and which are connectable hydraulically to patient feed line 4 and connecting tube 12 respectively by means of respective threaded connections 75, 76. Male member 60 and female member 61 are connectable releasably along axis 65 to connect conduits 62 and 63 hydraulically.

More specifically, male member 60 comprises a casing 67 made of rigid plastic and defining first conduit 62 and, outside first conduit 62, a seat 68 for releasably receiving a cylindrical end portion 61a of female member 61.

More specifically, casing 67 defines, on diametrically opposite sides of axis 65, two flexible circumferential lances 72 having respective teeth 69 on their free ends. Lances 72 flex outwards to permit insertion of end portion 61a of female member 61 inside seat 68, and, once end portion 61a is inserted, click back into the undeformed position so that teeth 69 rest against an annular shoulder 70 defining end portion 61a on the side facing threaded connection 76, thus preventing withdrawal of female member 61 from male member 60 along axis 65.

Male and female members 60, 61 are released using a pair of tabs 72a integral with respective lances 72, projecting outwards from casing 67, and for outwardly flexing lances 72.

Female member 61 is fitted, inside end portion 61a, with a cylindrical sealing member 73 made of elastomeric material, e.g. silicone, and having an axial through slit 73a.

Slit 73a is normally closed by the radial pressure exerted on sealing member 73 when force-fitted inside end portion 61a of female member 61, and is parted by insertion of first conduit 62 of male member 60, which, for the purpose, is tapered towards its free end. Once inserted, first conduit 62 extends through sealing member 73, along slit 73a, and is connected hydraulically to second conduit 63 of female member 61.

The advantages of the present invention will be clear from the foregoing description.

In particular, the manifold device can be inserted at any point along an intravenous infusion line, and permits connection of a desired number of fluid sources (in particular, a battery of drugs) for injection into the patient, or a desired number of withdrawal devices.

By means of the manifold device, all the tasks required during a surgical operation - i.e. controlled, selective drug administration with no risk of drug contamination, and withdrawal of blood from the patient - can be performed quickly and easily by simply rotating the selection knob.

Combined with non-return valve 80, the special releasable connecting device separating the patient feed line from the drug battery provides for separating the drug line in fluidtight manner, so that the drug line can safely be replaced from one patient to another.

Finally, the particular configuration of the infusion system provides for high infusion flow.

Clearly, changes may be made to the intravenous infusion system as described and illustrated herein without, however, departing from the scope of the present invention as defined in the accompanying Claims.

In particular, though three accesses are referred to in the description, the manifold device according to the invention may comprise any number of accesses.

The manifold device may be located at a point along the infusion line other than the one shown and described.

Finally, non-return valve 80 may be located at any point along patient feed line 4, and in particular may even be incorporated in male member 60.

## Claims

1. An intravenous infusion system (1) comprising a fluid infusion line (4, 12, 2) for feeding patients (3); and a manifold device (6) located along said infusion line (4, 12, 2) and having a first and a second opening (23, 25) connected hydraulically to said infusion line (4, 12, 2), and at least one access (7) connectable hydraulically to fluid administration/withdrawal means (8); said manifold device (6) also having valve means (35) defining a first one-way flow operating condition, in which only fluid flow from said administration/withdrawal, means (8) to said infusion line (4, 12, 2) is permitted;
wherein said valve means (35) can be set selectively to a second two-way flow operating condition, in which the valve means (35) permit fluid flow from said administration/withdrawal means (8) to said infusion line (4, 12, 2), and from said infusion line (4, 12, 2) to said administration/withdrawal means (8).

2. A system as claimed in Claim 1, **characterized in that** said first and said second operating condition of said valve means (35) permit distinct flow quantities.

3. A system as claimed in Claim 1 or 2, **characterized by** comprising non-return valve means (80) located along said infusion line (4, 12, 2), downstream from said manifold device (6), and for preventing fluid flow to the manifold device (6).

4. A system as claimed in any one of the foregoing Claims, wherein said valve means (35) comprise manually operated selecting means (37) for setting said valve means (35) to said first and said second operating condition.

5. A system as claimed in any one of the foregoing Claims, wherein said manifold device (6) comprises an elongated body (20) extending along a longitudinal axis (21) and defining a tubular main fluid flow conduit (22) having said first and said second opening (23, 25) and at least one access opening (30); and at least one tubular secondary conduit (31) extending from said access opening (30) along a first axis (29) crosswise to said longitudinal axis (21); said secondary conduit (31) defining said access (7) and being fitted with said valve means (35).

6. A system as claimed in Claim 5, wherein said secondary conduit (31) defines a cavity (33a) housing said valve means (35) and communicating, on opposite sides with respect to said first axis (29), with an open first portion (31a) of said secondary conduit (31) defining said access (7), and with an open second portion (31b) of said secondary conduit (31) defining said access opening (30).

7. A system as claimed in Claim 6, wherein said valve means (35) comprise a Tubular flexible shutter (40) inserted inside said cavity (33a), coaxially with a second axis (34) crosswise to said first axis (29) and to said longitudinal axis (21), so as to be interposed between said first and said second portion (31a, 31b); the length of said shutter (40) along said second axis (34) being less than the length of said cavity (33a), so as to define a free portion (44) of said cavity (33a).

8. A system as claimed in Claim 7, wherein said shutter (40) comprises four recesses (52a-52d) equally spaced angularly about said second axis (34), three of which (52a-52c) being formed in the outer lateral surface of said shutter (40), and the fourth (52d) of which being formed in the inner lateral surface of said shutter (40); said recesses (52a-52d) being aligned, in diametrically opposite pairs with respect to said second axis (34), with said first and said second portion (31a, 31b) in said first and said second operating condition; and said fourth recess (52d) being aligned with said first portion (31a) in said first operating condition.

9. A system as claimed in Claim 8, wherein said shutter (40) is integral with said selecting means (37); and said first and said second operating condition are determined by 90° rotation of said selecting means (37) about said second axis (34).

10. A system as claimed in any one of Claims 7 to 9, wherein said selecting means (37) axially engage said cavity (33a), and comprise a user grip portion (46); and a shaft (47) projecting from said grip portion (46) and externally supporting said shutter (40).

11. A system as claimed in any one of the foregoing Claims, wherein said infusion line (4, 12, 2) comprises at least one primary-fluid inlet line (2) and a patient feed line (4), said manifold device (6) being interposed between said inlet line (2) and said patient feed line (4); said system (1) also comprising a releasable connecting device (14) interposed between said manifold device (6) and said patient feed line (4).

12. A system as claimed in Claim 11, wherein said releasable connecting device (14) comprises a male member (60) and a female member (61), which define an elongated tubular first and second conduit (62, 63) extending along a common connection axis (65) and communicating hydraulically with respective portions of said infusion line (4, 12, 2); said female member (61) comprising an end portion (61a), and an elastically flexible sealing member (73) having an axial through slit (73a) and force-fitted inside said end portion (61a) so as to keep said slit (73a) normally closed; said slit (73a) being parted by insertion of said first conduit (62) when connecting said female member (61) and said male member (60).

13. A system as claimed in Claim 12, wherein said male member (60) comprises a casing (67) defining a seat (68) for releasably housing said end portion (61a) of said female member (61); and releasable retaining means (69, 72, 72a) which click onto said end portion (61a).

## Patentansprüche

1. Intravenöses Infusionssystem (1) mit einer Fluidinfusionsleitung (4, 12, 2) zum Versorgen von Patienten (3); einer Verteilungsvorrichtung (6), die entlang der Infusionsleitung (4, 12, 2) angeordnet ist und eine erste und eine zweite Öffnung (23, 25), die hydraulisch mit der Infusionsleitung (4, 12, 2) verbunden sind, und wenigstens einen Anschluss (7) aufweist, der hydraulisch mit einem Fluidverabreichungs/entnahmemittel (8) verbindbar ist; wobei die Verteilungsvorrichtung (6) auch ein Ventilmittel (35) aufweist, welches einen ersten Einwegflussbetriebszustand definiert, in welchem ein Fluidfluss nur von dem Verabreichungs/Entnahmemittel (8) zu der Infusionsleitung (4, 12, 2) erlaubt ist;
wobei das Ventilmittel (35) wahlweise auf einen zweiten Zweiwegeflussbetriebszustand eingestellt werden kann, in welchem das Ventilmittel (35) einen Fluidfluss von dem Verabreichungs/Entnahmemittel (8) zu der Infusionsleitung (4, 12, 2) und von der Infusionsleitung (4, 12, 2) zu dem Verabreichungs/Entnahmemittel (8) erlaubt.

2. System wie in Anspruch 1 beansprucht, **dadurch gekennzeichnet, dass** der erste und zweite Betriebszustand des Ventilmittels (35) unterschiedliche Flussmengen erlaubt.

3. System wie in Anspruch 1 oder 2 beansprucht, **gekennzeichnet durch** ein Aufweisen eines Sperrventilmittels (80), welches entlang der Infusionsleitung (4, 12, 2) nachgeordnet zu der Verteilungsvorrichtung (6) angeordnet ist, und zum Verhindern eines Fluidflusses zu der Verteilungsvorrichtung (6) ausgestaltet ist.

4. System wie in einem der vorstehenden Ansprüche beansprucht, wobei das Ventilmittel (35) ein manuell betreibbares Auswählmittel (37) zum Einstellen des Ventilmittels (35) auf den ersten und zweiten Betriebszustand aufweist.

5. System wie in einem der vorstehenden Ansprüche beansprucht, wobei das Verteilungsmittel (6) einen länglichen Körper (20) aufweist, der sich entlang einer Längsachse (21) erstreckt und eine rohrförmige Hauptfluidflussleitung (22) mit der ersten und der zweiten Öffnung (23, 25) definiert, und wenigstens eine Anschlussöffnung (30) aufweist; und wenigstens eine rohrförmige zweite Leitung (31) aufweist, die sich von der Anschlussöffnung (30) entlang einer ersten Achse (29) kreuzweise zu der Längsachse (21) erstreckt; wobei die zweite Leitung (31) den Anschluss (7) definiert und mit dem Ventilmittel (35) ausgestattet ist.

6. System wie in Anspruch 5 beansprucht, wobei die zweite Leitung (31) einen Hohlraum (33a) definiert, in dem das Ventilmittel (35) angeordnet ist und der an gegenüberliegenden Seiten bezüglich der ersten Achse (29) mit einem offenen ersten Teil (31 a) der zweiten Leitung (31), die den Anschluss (7) definiert, und mit einem offenen zweiten Teil (31 b) der zweiten Leitung (31) kommuniziert, die die Anschlussöffnung (30) definiert.

7. System wie in Anspruch 6 beansprucht, wobei das Ventilmittel (35) einen rohrförmigen flexiblen Verschluss (40) aufweist, der innerhalb des Hohlraumes (33a) koaxial mit einer zweiten Achse (34) kreuzweise zu der ersten Achse (29) und zu der Längsachse (21) angeordnet ist, um zwischen den ersten und zweiten Teilen (31 a, 31 b) angeordnet zu sein; wobei die Länge des Verschlusses (40) entlang der zweiten Achse (34) weniger als die Länge des Hohlraumes (33a) ist, um einen freien Teil (44) des Hohlraumes (33a) zu definieren.

8. System wie in Anspruch 7 beansprucht, wobei der Verschluss (40) vier Ausnehmungen (52a bis 52d) aufweist, die in gleichen Winkeln beabstandet um die zweite Achse (34) angeordnet sind, von denen drei (52a bis 52c) in der äußeren Seitenoberfläche des Verschlusses (40) gebildet sind, und wobei die vierte (52d) von ihnen in der inneren Seitenoberfläche des Verschlusses (40) gebildet ist; wobei die Ausnehmungen (52a bis 52d) bezüglich der zweiten Achse (34) in diametral gegenüberliegenden Paaren mit den ersten und zweiten Teilen (31a, 31b) in den ersten und zweiten Betriebszuständen ausgerichtet sind; und wobei die vierte Ausnehmung (52d) mit dem ersten Teil (31a) in dem ersten Betriebszustand ausgerichtet ist.

9. System wie in Anspruch 8 beansprucht, wobei der Verschluss (40) in das Auswählmittel (37) integriert ist; und wobei die ersten und zweiten Betriebszustände durch eine 90°-Rotation des Auswählmittels (37) um die zweite Achse (34) bestimmt sind.

10. System wie in einem von Ansprüchen 7 bis 9 beansprucht, wobei das Auswählmittel (37) axial mit dem Hohlraum (33a) in Eingriff steht und einen Verwendergriffteil (46); und einen Schaft (47) aufweist, der von dem Griffteil (46) absteht und den Verschluss (40) extern unterstützt.

11. System wie in einem der vorstehenden Ansprüche beansprucht, wobei die Infusionsleitung (4, 12, 2) wenigstens eine Primärfluideinlassleitung (2) und eine Patientenzuführungsleitung (4) aufweist, wobei die Verteilungsvorrichtung (6) zwischen der Einlassleitung (2) und der Patientenversorgungsleitung (4) angeordnet ist, wobei das System auch eine lösbare Verbindungsvorrichtung (14) aufweist, die zwischen der Verteilungsvorrichtung (6) und der Patientenversorgungsleitung (4) angeordnet ist.

12. System wie in Anspruch 11 beansprucht, wobei die lösbare Verbindungsvorrichtung (14) ein Steckerelement (60) und ein Buchsenelement (61) aufweist, welche eine längliche rohrförmige erste und zweite Leitung (62, 63) definieren, die sich entlang einer gemeinsamen Verbindungsachse (65) erstreckt und hydraulisch mit entsprechenden Teilen der Infusionsleitung (4, 12, 2) kommuniziert; wobei das Buchsenelement (61) ein Endteil (61 a) und ein elastisch flexibles Dichtelement (73) aufweist, welches einen axialen durchlaufenden Schlitz (73a) aufweist und kraftschließend innerhalb des Endteiles (61 a) angeordnet ist, um den Schlitz (73a) normalerweise geschlossen zu halten; wobei der Schlitz (73a) durch Einführung der ersten Leitung (62) geöffnet wird, wenn das Buchsenelement (61) und das Steckerelement (60) miteinander verbunden werden.

13. System wie in Anspruch 12 beansprucht, wobei das Steckerelement (60) ein Gehäuse (67), welches einen Sitz (38) zum lösbaren Umfassen des Endteiles (61a) des Buchsenelementes (61) aufweist; und ein lösbares Haltemittel (69, 72, 72a) aufweist, welches an dem Endteil (61 a) einrastet.

## Revendications

1. Système de perfusion intraveineuse (1) comprenant une ligne de perfusion de fluide (4, 12, 2) destinée à l'administration aux patients (3) et un dispositif collecteur (6) disposé le long de ladite ligne de perfusion (4, 12, 2) et comportant une première et une seconde ouverture (23, 25) reliées hydrauliquement à ladite ligne de perfusion (4, 12, 2) et au moins un accès (7) pouvant être connecté hydrauliquement à des moyens d'administration/extraction de fluides (8), ledit dispositif collecteur (6) comportant également des moyens du type valve (35) définissant un premier état de fonctionnement à écoulement unidirectionnel, dans lequel seul l'écoulement de fluide allant desdits moyens d'administration/extraction (8) à ladite ligne de perfusion (4, 12, 2) est permis ;
dans lequel lesdits moyens du type valve (35) peuvent être mis sélectivement dans un second état de fonctionnement bidirectionnel dans lequel les moyens du type valve (35) permettent l'écoulement du fluide allant desdits moyens d'administration/extraction (8) à ladite ligne de perfusion (4, 12, 2) et allant de ladite ligne de perfusion (4, 12, 2) auxdits moyens d'administration/extraction (8).

2. Système tel que revendiqué dans la revendication 1, **caractérisé en ce que** lesdits premier et second états de fonctionnement desdits moyens du type valve (35) autorisent des quantités d'écoulement distinctes.

3. Système tel que revendiqué dans la revendication 1 ou 2, **caractérisé en ce qu'**il comprend des moyens du type valve anti-retour (80) disposés le long de ladite ligne de perfusion (4, 12, 2), en aval dudit dispositif collecteur (6) et destinés à empêcher l'écoulement du fluide allant au dispositif collecteur (6).

4. Système tel que revendiqué dans l'une quelconque des revendications précédentes, dans lequel lesdits moyens du type valve (35) comprennent des moyens de sélection (37) actionnés manuellement pour mettre lesdits moyens du type valve (35) dans lesdits premiers et second états de fonctionnement.

5. Système tel que revendiqué dans l'une quelconque des revendications précédentes, dans lequel ledit dispositif collecteur (6) comprend un corps allongé (20) s'étendant selon un axe longitudinal (21) et définissant un conduit tubulaire principal d'écoulement de fluide (22) qui comporte lesdites première et seconde ouvertures (23, 25) et au moins une ouverture d'accès (30), et au moins un conduit tubulaire secondaire (31) qui s'étend à partir de ladite ouverture d'accès (30) le long d'un premier axe (29) transversalement audit axe longitudinal (21), ledit conduit secondaire (31) définissant ledit accès (7) et étant équipé desdits moyens du type valve (35).

6. Système tel que revendiqué dans la revendication 5, dans lequel ledit conduit secondaire (31) définit une cavité (33a) qui renferme lesdits moyens du type valve (35) et qui communique, sur des côtés opposés par rapport audit premier axe (29), avec une première partie ouverte (31 a) dudit conduit secondaire (31) définissant ledit accès (7) et avec une seconde partie ouverte (31 b) dudit conduit secondaire (31) définissant ladite ouverture d'accès (30).

7. Système tel que revendiqué dans la revendication 6, dans lequel lesdits moyens du type valve (35) comprennent un obturateur flexible tubulaire (40) inséré dans ladite cavité (33a), coaxialement à un second axe (34) transversalement audit premier axe (29) et audit axe longitudinal (21), de manière à être interposé entre lesdites première et seconde parties (31 a, 31 b), la longueur dudit obturateur (40) selon ledit second axe (34) étant plus petite que la longueur de ladite cavité (33), de manière à définir une partie libre (44) de ladite cavité (33a).

8. Système tel que revendiqué dans la revendication 7, dans lequel ledit obturateur (40) comprend quatre cavités (52a-52d) uniformément espacées angulairement autour dudit second axe (34), dont trois (52a-52c) sont formées dans la surface latérale extérieure dudit obturateur (40), la quatrième (52d) étant formée dans la surface latérale intérieure dudit obturateur (40), lesdites cavités (52a-52d) étant alignées, en paires diamétralement opposées par rapport audit second axe (34), avec lesdites première et seconde parties (31a, 31 b) dans lesdits premier et second états de fonctionnement, et ladite quatrième cavité (52d) étant alignée avec ladite première partie (31 a) dans ledit premier état de fonctionnement.

9. Système tel que revendiqué dans la revendication 8, dans lequel ledit obturateur (40) est réalisé d'une pièce avec lesdits moyens de sélection (37), et lesdits premier et second états de fonctionnement sont déterminés par une rotation de 90° desdits moyens de sélection (37) autour dudit second axe (34).

10. Système tel que revendiqué dans l'une quelconque des revendications 7 à 9, dans lequel lesdits moyens de sélection (37) entrent en prise axialement avec ladite cavité (33a) et comprennent une partie de prise pour l'utilisateur (46) et une tige (47) qui fait saillie sur ladite partie de prise (46) et supporte extérieurement ledit obturateur (40).

11. Système tel que revendiqué dans l'une quelconque des revendications précédentes, dans lequel ladite ligne de perfusion (4, 12, 2) comprend au moins une ligne d'entrée de fluide principal (2) et une ligne d'administration au patient (4), ledit dispositif collecteur (6) étant interposé entre ladite ligne d'entrée (2) et ladite ligne d'administration au patient (4), ledit système (1) comprenant en outre un dispositif de raccordement détachable (14) interposé entre ledit dispositif collecteur (6) et ladite ligne d'administration au patient (4).

12. Système tel que revendiqué dans la revendication 11, dans lequel ledit dispositif de raccordement détachable (14) comprend un élément mâle (60) et un élément femelle (61), qui définissent des premier et second conduits tubulaires allongés (62, 63) s'étendant le long d'un axe de raccordement commun (65) et qui communiquent hydrauliquement avec des parties respectives de ladite ligne de perfusion (4, 12, 2), ledit élément femelle (6) comprenant une partie d'extrémité (61 a), et un élément de fermeture étanche élastiquement flexible (73) ayant une fente traversante axiale (73a) et emmanché à force dans ladite partie d'extrémité (61 a) de manière à maintenir ladite fente (73a) normalement fermée; ladite fente (73a) étant ouverte par l'insertion dudit premier conduit (62) lorsqu'il raccorde ledit élément femelle (61) audit élément mâle (60).

13. Système tel que revendiqué dans la revendication 12, dans lequel ledit élément mâle (60) comprend un boîtier (67) définissant un siège (68) destiné à loger de façon détachable ladite partie d'extrémité (61 a) dudit élément femelle (61) et des moyens de retenue détachables (69, 72, 72a) qui s'encliquettent sur ladite partie d'extrémité (61 a).
